**Europäisches Patentamt**

**European Patent Office**

**Office européen des brevets**

(19)

(11) Veröffentlichungsnummer : **0 174 652 B1**

(12) **EUROPÄISCHE PATENTSCHRIFT**

(45) Veröffentlichungstag der Patentschrift :
28.03.90

(21) Anmeldenummer : 85111492.6

(22) Anmeldetag : 11.09.85

(51) Int. Cl.⁵ : **G 01 N 33/543,**
**G 01 N 33/563,**
**G 01 N 33/577**

(54) Immunchemisches Messverfahren für Haptene und Proteine.

(30) Priorität : 13.09.84 DE 3433652

(43) Veröffentlichungstag der Anmeldung :
19.03.86 Patentblatt 86/12

(45) Bekanntmachung des Hinweises auf die Patenterteilung : 28.03.90 Patentblatt 90/13

(84) Benannte Vertragsstaaten :
AT BE CH DE FR GB IT LI LU NL SE

(56) Entgegenhaltungen :
DE--B-- 2 155 658
DE--B-- 2 743 444
US--A-- 4 056 608

(73) Patentinhaber : BOEHRINGER MANNHEIM GMBH
Patentabteilung, Abt. E Sandhofer Strasse 112-132
Postfach 31 01 20
D-6800 Mannheim 31 Waldhof (DE)

(72) Erfinder : Baier, Manfred, Dr. rer. nat.
Tiefentalweg 10
D-8124 Seeshaupt (DE)
Erfinder : Jering, Helmut, Dr. rer. nat.
Anton-Bartl-Strasse 4
D-8132 Tutzing (DE)
Erfinder : Klose, Sigmar, Dr. phil.
Breitenloh 7
D-8131 Berg 2 (DE)

(74) Vertreter : Weickmann, Heinrich, Dipl.-Ing. et al
Patentanwälte Dipl.-Ing. H.Weickmann Dipl.-Phys.Dr.
K.Fincke Dipl.-Ing. F.A.Weickmann Dipl.-Chem. B.
Huber Dr.-Ing. H. Liska Dipl.-Phys.Dr. J. Prechtel
Postfach 860820
D-8000 München 86 (DE)

**Beschreibung**

Die Erfindung betrifft ein Verfahren zur immunologischen Bestimmung einer antigen wirksamen Substanz und ein hierfür geeignetes Reagenz.

Die Entwicklung der Immuntests hat, ausgehend vom Radioimmunverfahren (RIA), zu einer entscheidenden Verbesserung der Empfindlichkeit und Selektivität der Bestimmung von antigen wirksamen Substanzen wie Proteinen und Haptenen geführt und insbesondere in den klinisch chemischen Laboratorien große Bedeutung erlangt. Durch die Weiterentwicklung dieser Methoden wurde neben der Verbreiterung der zur Verfügung stehenden Markierungsmethoden, bei welchen neben einer radioaktiven Markierung vor allem die Enzymmarkierung (EIA) besondere Bedeutung erlangt hat, auch die Entwicklung der Umsetzungstechniken erheblich differenziert. So wurden sowohl in homogener als auch in heterogener Phase ablaufende Tests entwickelt, obwohl bei letzteren die in der Regel erforderliche Trennung von fester und flüssiger Phase zu Verschleppungs- und Verdünnungsproblemen führen kann.

Für ein derartiges Verfahren besonders erwünscht ist dabei ein Meßsignal, welches der Menge der zu bestimmenden antigen wirksamen Substanz, im foldenden vereinfachend als Antigen bezeichnet, direkt proportional ist. Weiter wird eine Verfahrensführung gewünscht, bei der eine Störung durch Komponenten der zu untersuchenden Lösung, insbesondere durch Serumkomponenten, mit Sicherheit vermieden wird. Schließlich soll das Verfahren sehr hohe Empfindlichkeit aufweisen und auch im Pikomolbereich, wie er insbesondere bei Haptenen häufig vorliegt, noch ausreichend genaue Ergebnisse liefern.

So ist es bereits bekannt, bei den aufgrund ihrer nur einen für Antikörper zugänglichen Bindungsstelle besondere Probleme aufwerfenden Haptenen die Bestimmung so durchzuführen, daß man eine exakt dosierte Menge von Antihaptenantikörper und von markiertem Hapten mit dem zu bestimmenden Hapten in der Probe um die Bindung am Antikörper konkurrieren läßt (DE-AS 21 55 658). Bei diesem Verfahren lassen sich zwar Störungen durch Fremdkomponenten ausschließen, aufgrund des kompetitiven Prinzips ist jedoch die Empfindlichkeit zu gering. Weiter ist das sogenannte IEMA-Verfahren bekannt, bei welchem Proteine durch Umsetzung mit einem markierten Antikörper, der in beliebigem Überschuß vorliegt und Abtrennung des nicht umgesetzten Antikörpers mit Hilfe von in fester Phase vorliegendem Protein sowie nachfolgender Messung der in der flüssigen Phase verbliebenen Menge an markiertem Antikörper durchgeführt wird (Lancet, August 1968, 492-493). Aus der DE-AS 27 43 444 ist es weiterhin bekannt, bei diesem IEMA-Verfahren anstelle des vollständigen Antikörpers Fab-Fragmente desselben zu verwenden und damit Haptene und Protein zu bestimmen. Der Nachteil dieser Verfahren besteht darin, daß Störungen durch Fremdsubstanzen möglich sind und der markierte Antikörper bzw. sein Fragment genau dosiert werden müssen.

Der Erfindung liegt daher die Aufgabe zugrunde, die obengenannten Bedingungen in einem Verfahren zu vereinen, welches die erwähnten Nachteile nicht mehr aufweist.

Insbesondere ist es eine Aufgabe der Erfindung, Serumbestandteile, die bei der Bestimmung nicht interessieren oder gar stören können, zu entfernen, die feste Phase ohne Empfindlichkeitsverlust auswaschen zu können und die Empfindlichkeitssteigerung zu erreichen.

Gelöst wird diese Aufgabe erfindungsgemäß durch ein Verfahren zur immunchemischen quantitativen Bestimmung einer antigen wirksamen Substanz durch Inkubation der zu bestimmenden Substanz mit einem Binder, der aus einem markierten, gegen die zu bestimmende Substanz gerichteten ersten Antikörper oder einem Fab- oder Fab'-Fragment davon besteht, Inkubieren des Umsetzungsgemisches für eine bestimmte Zeitdauer mit an eine feste Phase gebunden vorliegender zu bestimmender Substanz oder einem Analogon davon, Abtrennung der flüssigen Phase, gegebenenfalls Waschen der festen Phase und Vereinigung der Waschflüssigkeit mit der flüssigen Phase, welches dadurch gekennzeichnet ist, daß man die flüssige Phase danach mit einem an eine feste Phase gebunden vorliegenden zweiten Antikörper, der gegen den Binder oder den Komplex aus Binder und zu bestimmender Substanz gerichtet ist und mit keiner der Komponenten des Komplexes kreuzreagiert wenn er gegen den Komplex gerichtet ist, kontaktiert, die feste Phase abtrennt und wäscht und die daran gebundene Markierung mißt.

Das erfindungsgemäße Verfahren weist überraschenderweise eine sehr hohe Genauigkeit auf, obwohl zwei verschiedene feste Phasen verwendet werden und daher zweimal eine Trennung von flüssiger und fester Phase erforderlich ist. Durch unspezifische Adsorption am Festkörper hätte man bei einem derartigen Verfahren durch Verschleppung eine die Brauchbarkeit ausschließende Ungenauigkeit erwartet. Dies gilt umso mehr, wenn zusätzliche Waschschritte nach Abtrennung der ersten festen Phase zu einer Verdünnung der flüssigen Phase führen.

Das Verfahren der Erfindung eignet sich insbesondere auch für die Bestimmung von kleinsten Mengen an Haptenen, also Substanzen, die an sich nicht antigen wirksam sind, jedoch durch Kopplung mit einer weiteren antigen wirksamen Substanz doch die Bildung von spezifischen Antikörpern hervorrufen können. Bisher konnten Haptene nach sogenannten « Sandwich »-Verfahren nicht bestimmt werden, da sie aufgrund ihres geringen Molekulargewichts nur eine einzige für Antikörper zugängliche Bindungsstelle besitzen. Das Verfahren der Erfindung eignet sich aber ebenso für die Bestimmung von anderen Antigenen, insbesondere von Proteinen und Allergenen.

Der beim erfindungsgemäßen Verfahren verwendete Binder besteht, wie oben bereits erwähnt, aus einem markierten ersten Antikörper, der gegen die zu bestimmende antigen wirksame Substanz gerichtet ist oder einem Fab- oder Fab'-Fragment davon. Der Binder kann im Rahmen der Erfindung in einem beliebigen Überschuß bezüglich der Menge der zu bestimmenden Substanz in der Probe eingesetzt werden. Es ist jedoch auch möglich, den Binder in einem molaren Unterschuß einzusetzen, wenn die Dauer der Inkubation mit der zu bestimmenden Substanz so kurz gewählt wird, daß noch ein nichtgebundener Anteil vorhanden ist, welcher mit der in fester Phase vorliegenden zu bestimmenden Substanz reagieren kann. Der erste Antikörper kann dabei ein polyklonaler oder ein monoklonaler Antikörper sein.

Als Marker eignen sich die üblicherweise im immunchemischen Verfahren gebräuchlichen Marker wie optisch bestimmbare Gruppen oder Verbindungen z. B. fluoreszierende oder gefärbte Substanzen, Farbentwicklungskomponenten und dergleichen, insbesondere aber radioaktive Atome und Verbindungen sowie Enzyme. Letztere werden bevorzugt aufgrund ihrer einfachen Bestimmbarkeit ohne besondere Apparaturen, wie sie bei radioaktiven Substanzen, Fluoreszenzmarkierungen und dergleichen erforderlich sind. Als Markierungsenzyme werden zweckmäßig die im Enzymimmunassay üblichen Enzyme wie Peroxidase, alkalische Phosphatase, β-Galactosidase, Glucoamylase, Glucoseoxidase, Acetylcholinesterase, Katalase, Lysozym, Malatdehydrogenase, Glucose-6-phosphat-Dehydrogenase und β-Amylase verwendet. Besonders bevorzugt werden Peroxidase aufgrund ihrer Stabilität und günstigen Bestimmungsmöglichkeit über gebildetes $H_2O_2$ sowie β-Galactosidase.

Die erste feste Phase, die im Rahmen der Erfindung verwendet wird, enthält gebundenes Antigen oder ein Analogon davon. Unter Analogon wird hier eine chemisch ähnliche Substanz, die mit dem ersten Antikörper kreuzreagiert, wenn auch meistens in geringerem Ausmaß als die zu bestimmende Substanz selbst, verstanden. Zweckmäßigerweise ist das Antigen mit einer kovalenten oder adsorptiven Bindung oder durch Präzipitation an ein geeignetes inertes Substrat gebunden. Diese Methoden sind dem Fachmann geläufig und bedürfen hier keiner näheren Erläuterung. Gleiches gilt auch für die in Betracht kommenden Trägermaterialien. In der Regel handelt es sich um Glas, Kunststoffe oder synthetische oder natürliche Polymere wie Zellulose, Dextrane, Styrolpolymerisate und dergleichen. Auch Ionenaustauscher und unspezifische Adsorber können verwendet werden. Diese Trägermaterialien sind dem Fachmann ebenfalls geläufig. Das in fester Phase vorliegende Antigen bedarf im Rahmen des erfindungsgemäßen Verfahrens keiner exakten Dosierung. Es muß nur ausreichen, um den Überschuß an Binder, der durch das zu bestimmende Antigen in der Probe nicht bereits gebunden ist, durch Antigen-Antikörper-Reaktion zu fixieren.

Auch der zweite Antikörper, welcher ebenfalls in unlöslicher Phase vorliegt, kann nach den oben erwähnten Methoden an die Träger gebunden vorliegen. Auch hier ist beim Verfahren der Erfindung keine exakte Dosierung notwendig. Es genügt, wenn der zweite Antikörper in ausreichender Menge vorhanden ist, um allen in der flüssigen Phase, welche von der in fester Phase vorliegenden, zu bestimmenden Substanz abgetrennt wurde, vorhandenen Komplex aus Binder und zu bestimmender Substanz sandwichartig zu fixieren. Dabei kann der zweite Antikörper gegen den ersten Antikörper, dessen Fab- oder Fab'-Fragment oder gegen dessen Markierung gerichtet sein. Alternativ kann der zweite Antikörper auch gegen den Komplex selbst gerichtet sein, den der Binder mit der zu bestimmenden Substanz bildet. In diesem Falle darf der zweite Antikörper weder mit dem Marker noch mit dem ersten Antikörper im gebildeten Komplex kreuzreagieren, d. h. er muß für die Bindung zwischen Binder und zu bestimmender Substanz spezifisch sein. Der zweite Antikörper bindet so den in der flüssigen Phase vorliegenden, von der ersten festen Phase nicht gebundenen Antigen-markierter Binder-Komplex und wird nach Abtrennung der flüssigen Phase und Auswaschen auf die trägergebundene Markierung vermessen. Dies erfolgt bei Enzymmarkierung einfach durch Kontakt mit einem flüssigen Reagenz zur Bestimmung des Markerenzyms nach den hierfür üblichen Methoden. Ist der Marker kein Enzym, so erfolgt seine Bestimmung ebenfalls nach einer der dafür bekannten und dem Fachmann geläufigen Methoden. Der zweite Antikörper kann polyklonal, monoklonal oder ein Fab/Fab'-Fragment davon sein.

Beim erfindungsgemäßen Verfahren wird, je nachdem, ob Binder im Überschuß oder im Unterschuß, bezogen auf die zu bestimmende Substanz zugegeben wird, bei der ersten Inkubation ein Reaktionsgemisch erhalten, welches aus Komplex zwischen Binder und zu bestimmender Substanz, restlichem freiem Binder und gegebenenfalls restlicher freier zu bestimmender Substanz besteht. Letzteres ist dann der Fall, wenn die Inkubation so kurz ist, daß nicht alle zu bestimmende Substanz in den Komplex eintritt und Binder im Unterschuß vorliegt.

Durch die zweite Inkubation wird an der festen Phase die Trennung dieses Reaktionsgemisches durchgeführt, da an diese Festphase nur freier Binder fixiert wird, nicht aber der im Komplex enthaltene Binder. Bei abschließender Phasentrennung geht daher der Komplex in die flüssige Phase über und wird aus letzterer mit dem zweiten, in fester Phase vorliegenden Antikörper, abgetrennt. An die zweite feste Phase fixierter Komplex enthält daher die Markierung des Binders in einer der zu bestimmenden Substanz direkt proportionalen Menge. Anschließend wird die Menge an Marker gemessen. Handelt es sich bei dem Marker beispielsweise um ein Enzym wie ß-Galactosidase oder Peroxidase, so erfolgt der Nachweis dieses Enzyms in der hierfür bekannten Weise. Analog erfolgt der Nachweis einer radioaktiven Markierung durch entsprechende Meßgeräte, einer optischen Markierung durch optische Geräte.

Das erfindungsgemäße Verfahren weist eine hohe Präzision auf mit einem Variationskoeffizienten, der in der Regel zwischen 5 und 10 % liegt bei Bestimmung von Haptenen als Antigenen im Konzentrationsbereich von etwa 1 ng/ml.

Das erfindungsgemäße Verfahren wird weder durch andere Bestandteile der zu untersuchenden Lösung, welche das Antigen enthält, gestört, was insbesondere die häufigen Störungen durch Serumkomponenten ausschließt, noch erfordert es eine exakte Dosierung der verschiedenen eingesetzten Komponenten wie Binder, in fester Phase vorliegendes Antigen und zweiter Antikörper. Darüberhinaus liefert das Verfahren einen Meßwert, welcher der vorhandenen Menge an zu bestimmendem Antigen direkt proportional ist.

Ein weiterer Gegenstand der Erfindung ist ein Reagenz zur immunchemischen Bestimmung einer antigen wirksamen Substanz, welches gekennzeichnet ist durch

a) einen Gehalt an einem Binder, der aus einem markierten, gegen die zu bestimmende Substanz gerichteten ersten Antikörper oder einem Fab- bzw. Fab'-Fragment davon besteht,

b) an eine feste Phase gebundenem Antigen,

c) an eine feste Phase gebundenem zweiten Antikörper, der gegen den Binder oder den Komplex aus Binder und zu bestimmender Substanz gerichtet ist und

d) einem System zur Messung der Markierungssubstanz.

In einer bevorzugten Ausführungsform enthält des erfindungsgemäße Reagenz einen enzymhaltigen Binder zusammen mit einem System zur Bestimmung dieses Enzyms.

Die folgenden Beispiele erläutern die Erfindung weiter.

Beispiel 1

Verfahren zur Bestimmung von Digoxin in Humanserum

1. Reagentien
Gewinnung des Antiserums gerichtet gegen Digoxin :

Die Herstellung des Immunogens, nämlich Humanserum-Albumin, konjugiert mit Digoxin, erfolgte so wie von V. P. Butler jr. und J. P. Chen, Proc. Nat. Acad. Sci. US 51, 71-78 (1967) sowie von T. W. Smith, V. P. Butler und E. E. Haber, Biochemistry 9, 331-337 (1970) ausführlich beschrieben. Mit diesem Immunogen wurden Schafe immunisiert und das entsprechende Antiserum gewonnen.

Gewinnung des Antiserums gegen β-Galactosidase :

Schafe wurden mit β-Galactosidase (Boehringer Mannheim, Best. Nr. 570 079) immunisiert und das entsprechende Antiserum gewonnen. Lit. : E.

Ishiskawa, S. Yoshitake in : « Enzyme Immunoassay » ; E. Ishikawa, T. Kawai, K. Miyai eds., Igaku-Shoin, Tokyo/New York (1981).

Gewinnung des Antiserums gerichtet gegen Kaninchen IgG :

Kaninchen-Schlachtserum wurde ad 1,8 M mit NH₄SO₄ versetzt. Das Präzipitat wird in einem Puffer aus 15 mM Natriumphosphat, pH 7,0 und 50 mM NaCl aufgenommen und die so erhaltene Lösung einer Passage über DEAE-Cellulose unterworfen. Die IgG enthaltenden Fraktionen wurden lyophilisiert und als Immunogen verwendet. Mit diesem Immunogen wurden Schafe immunisiert und das entsprechende Antiserum gewonnen.

Herstellung von Festphase 1 :

Kopplung von Digoxin an Kaninchen-IgG :
Kaninchen-IgG wurde wie oben beschrieben gewonnen. Digoxin wurde mit Natrium-meta-perjodat oxidiert und an Kaninchen-IgG gekoppelt. Das Verfahren und die technischen Einzelheiten sind ausführlich von V. P. Butler jr. und J. P. Chen, Proc. Nat. Acad. Sci. US, 57, 7 (1967) sowie von T. W. Smith et al., Biochem. 9, 331-337 (1970) beschrieben. Abweichend von diesen beiden Vorschriften wurde als Trägerprotein nicht Serum-Albumin, sondern über DEAE-Cellulose aufgereinigtes Kaninchen-IgG verwendet. Mit Digoxin derivatisiertes Kaninchen IgG wurde an das « Affinitätsadsorbens, Glutardialdehyd aktiviert » von Boehringer Mannheim (Best. Nr. 665 525) nach der Arbeitsvorschrift des Herstellers gekoppelt.

Herstellung von Festphase 2 :

Die Herstellung erfolgt durch Kopplung von anti-β-Galactosidase IgG-Fraktion an das « Affinitätsadsorbens, Glutardialdehyd aktiviert » von Boehringer Mannheim (Best. Nr. 665 525) nach der Arbeitsvorschrift des Herstellers. Die IgG-Fraktionen wurden aus den entsprechenden Antiseren über Ammonsulfat-Fällung und Chromatographie über DEAE-Cellulose gewonnen.

Herstellung des Binders :

Antiserum, gerichtet gegen Digoxin, wurde über Ammoniumsulfat-Fällung und Passage über DEAE-Cellulose zur IgG-Fraktion aufgereinigt. Die Papain-Spaltung wurde nach R. R. Porter, Biochem. J. 73, 119-126 (1959) durchgeführt. Die Fab-Fragmente wurden von den nicht verdauten IgG-Molekülen und den Fc-Fragmenten mittels Gelfiltration über Sephadex G 100 und Ionenaustausch-Chromatographie über DEAE-Cellulose nach Literaturvorschrift (K. Malinowski und W. Manski in « Methods in Enzymology » ; J. J. Langone und H. van Vunakis eds., Academic Press, Vol. 73 (1981), 418-459) abgetrennt. Die resultierende Fab-Fraktion wurde über eine Säule, beschickt mit Festphase 1, geschickt. Der erste Elutionspuffer war 0,1 M Natriumphosphat, pH 7,2 plus 0,9 M NaCl. Nach Elution der unspezifischen, d. h. nicht gegen Digoxin gerichteten Fab-Fragmente, wurden die gegen Digoxin gerichteten Fab-Fragmente mit 1 M Proprionsäure eluiert. Das Eluat wurde anschließend gegen Wasser dialysiert, durch Ultrafiltration eingeengt und lyophilisiert. Die gegen Digoxin gerichteten Fab-Fragmente wurden mit dem hetero-bifunktionellen Reagens N-(-m-malein-imido-benzoyloxy) succinimid (MBS) umgesetzt und anschließend an β-Galactosidase nach der Vorschrift von T. Kitiwaga in « Enzyme Immunoassay » ; Ishikawa, T. Kawai, K. Miyai, eds., Igaku Shoin, Tokyo/New York (1981) gekoppelt. Nach Kopplung wurde das Fab-β-Gal-Konjugat einer Gelfiltration über Sepharose 6 B, wie von den eben genannten Autoren beschrieben, unterzogen. Verwendet wurden die Fraktionen, die keine freie, d. h. ungekoppelte β-Galactosidase, enthielten.

## 2. Bestimmung von Digoxin

Digoxin-Standards in Humanserum (entnommen aus dem Elisa-Kit von Boehringer Mannheim, Best. Nr. 199 656) wurden 1 : 7,5 mit 0,9 % NaCl-Lösung verdünnt.

Zu 200 µl verdünntem Standard wurde 200 µl Binder (20 mU/ml, bestimmt mit ortho-nitrophenyl-β-D-galactosid), hergestellt wie unter 3.7 beschrieben, gegeben. Der Binder ist in folgendem Puffer gelöst :

0,1 M Natriumphosphat pH 7,2
0,2 % NaCl
2 mM $MgCl_2$
1 % Rinder-Serum-Albumin

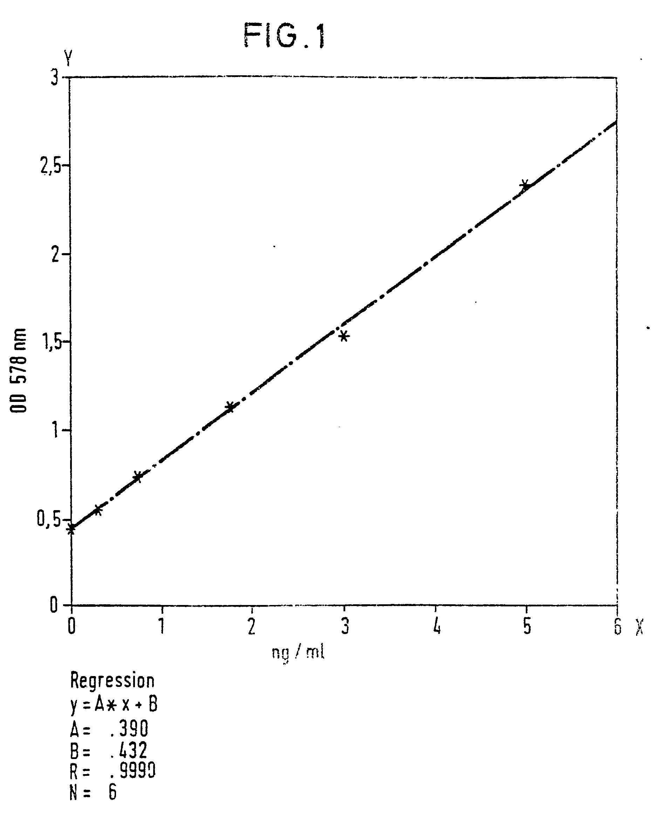

Standard und Binder werden genau 5 min bei 37 °C inkubiert. Danach erfolgt die Zugabe von 50 µl Festphase 1. Das Gemisch wird weitere 5 min unter Schütteln bei 37 °C inkubiert. Anschließend wird 1 min mit einer Eppendorf-Zentrifuge 54/4 zentrifugiert. Vom Überstand werden 300 µl entnommen, hinzugefügt wird 50 µl Festphase 2 und unter Schütteln bei 37 °C inkubiert. Der Ansatz wird nach Ablauf dieser 5 min zentrifugiert, der Überstand verworfen und das Pellet nochmals 2 × mit dem oben angegebenen Puffer gewaschen. Zu dem gewaschenen Pellet werden 0,6 ml einer 6 mM Lösung an Chlorphenolrot-β-Galactosid (Herstellung siehe Deutsche Patentanmeldung P 33 45 748) gelöst in 0,1 M Natriumphosphatpuffer, pH 7,2, 0,9 % NaCl, 2 mM $MgCl_2$ und Lösung suspendiert und 5 min unter Schütteln (37 °C) inkubiert. Danach wird wie oben abzentrifugiert. Vom Überstand werden 0,5 ml entnommen und davon die optische Dichte bei 578 nm bestimmt. Figur 1 der Zeichnung zeigt den Verlauf einer so erhaltenen Eichkurve. Aufgetragen wurde die Digoxin-Konzentration des unverdünnten Serums (X-Achse) gegen die dazugehörige optische Dichte (Y-Achse).

## Beispiel 2

Verfahren zur Bestimmung von Digoxin mit Hilfe eines gegen Digoxin gerichteten monoklonalen Antikörpers

## 1. Reagentien

Es werden dieselben Reagentien wie im Beispiel 1 verwendet bzw. hergestellt mit Ausnahme der Herstellung von Festphase 2 und des Binders.

Gewinnung des Antiserums gerichtet gegen Maus-IgG :

Maus-Serum wurde ad 1,8 M $NH_4SO_4$ versetzt. Das Präzipitat wird in einem Puffer aus 15 mM Natriumphosphat, pH 7,0 und 50 mM NaCl aufgenommen und die so erhaltene Lösung einer Passage über DEAE-Cellulose unterworfen. Die IgG enthaltenden Fraktionen wurden lyophilisiert und als Immunogen verwendet. Mit diesem Immunogen wurden Schafe immunisiert und das entsprechende Antiserum gewonnen.

Herstellung der Festphase 2 :

Schaf-IgG, gegen Maus-IgG gerichtet, wurde an « Affi-Gel 10 » (Amicon) nach der Arbeitsvorschrift des Herstellers gekoppelt. Kopplungsbedingungen : 5 h bei 4 °C in Natriumphosphat-Puffer, pH 7,5, Protein-Konzentration 8 mg/ml, anschließend 3 h Raumtemperatur unter Zusatz von 0,1 M Äthanolamin, pH 8,0.

Herstellung des Binders :

Monoklonale Antikörper gegen Digoxin werden nach der Methode von Köhler und Milstein, Eur. J. Immunol. 6, 292 (1976) hergestellt. Dieser monoklonale Antikörper gegen Digoxin enthaltende Ascites wurde über Ammoniumsulfat-Fällung und Passage über DEAE-Cellulose zur IgG-Fraktion aufgereinigt. Die Papain-Spaltung wurde nach R. R. Porter, Biochem. J. 73, 119-126 (1959) durchgeführt. Die Fab-Fragmente wurden von den nicht verdauten IgG-Molekülen und den Fc-Fragmenten mittels Gelfiltration über Sephadex G 100 und Ionenaustausch-Chromatographie über DEAE-Cellulose nach Literaturvorschrift (K. Malinowski und W. Manski in « Methods in Enzymology » ; J. J. Langone und H. van Vunakis eds., Academic Press, Vol. 73 (1981), 418-459) abgetrennt. Die resultierende Fab-Fraktion wurde über eine Säule, beschickt mit Festphase 1, die wie unter Punkt 3.5.3 hergestellt wurde, geschickt. Der erste Elutionspuffer war 0,1 M Natriumphosphat, pH 7,2 plus 0,9 M NaCl. Nach Elution der unspezifischen, d.h. nicht gegen Digoxin gerichteten Fab-Fragmente, wurden die gegen Digowin gerichteten Fab-Fragmente mit 1 M Proprionsäure eluiert. Das Eluat wurde anschließend gegen Wasser dialysiert, durch Ultrafiltration eingeengt und lyophilisiert. Die gegen Digoxin gerichteten Fab-Fragmente wurden mit dem hetero-bifunktionellen Reagens N-(-m-malein-imido-benzoyloxy) succinimid (MBS) umgesetzt und anschließend an β-Galactosidase nach der Vorschrift von T. Kitiwaga in « Enzyme Immunoassay » ; Ishikawa, T. Kawai, K. Miyai, eds. Igaku Shoin, Tokyo/New York (1981) 81-89, gekoppelt. Nach Kopplung wurde das Fab-β-Gal-Konjugat einer Gelfiltration über Sepharose 6 B wie von den eben genannten Autoren beschrieben unterzogen. Verwendet wurden die Fraktionen, die keine freie, d. h. ungekoppelte β-Galactosidase, enthielten.

## 2. Bestimmung von Digoxin

Digoxin-Standards in Humanserum (entnommen aus dem Elisa-Kit von Boehringer Mannheim, Best. Nr. 199 656) wurden 1 : 7,5 mit 0,9 % NaCl-Lösung verdünnt. Zu 200 µl verdünntem Standard wurde 200 µl Binder (20 mU/ml, bestimmt mit ortho-nitrophenyl-β-D-galactosid), hergestellt wie unter 3.7 beschrieben, gegeben. Der Binder ist in folgendem Puffer gelöst :

0,1 M Natriumphosphat pH 7,2
0,2 % NaCl
2 mM MgCl$_2$
1 % Rinder-Serum-Albumin

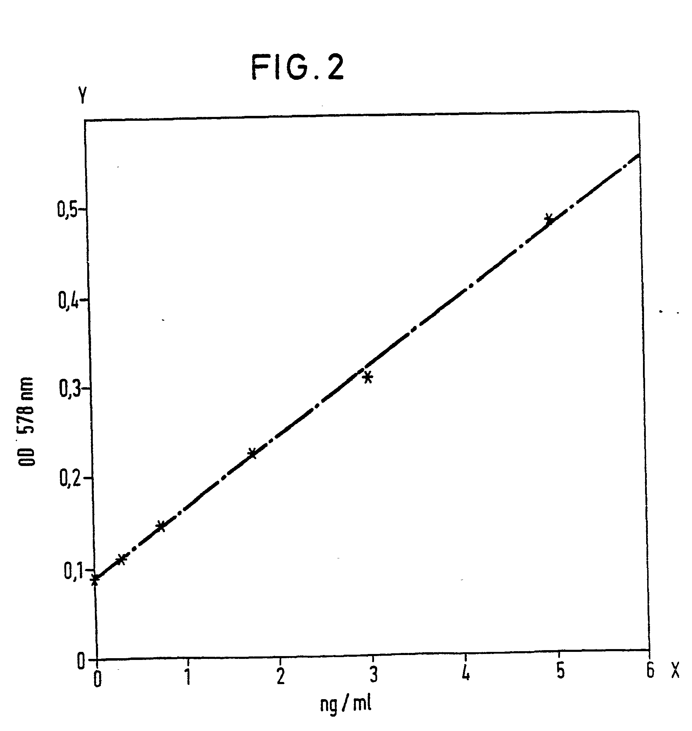

Standard und Binder werden genau 5 min bei 37 ˚C inkubiert. Danach erfolgt die Zugabe von 50 µl Festphase 1. Das Gemisch wird weitere 5 min unter Schütteln bei 37 ˚C inkubiert. Anschließend wird 1 min mit einer Eppendorf-Zentrifuge 54/4 zentrifugiert. Vom Überstand werden 300 µl entnommen, hinzugefügt wird 50 µl Festphase 2 und unter Schütteln bei 37 ˚C inkubiert. Der Ansatz wird nach Ablauf dieser 5 min zentrifugiert, der Überstand verworfen und das Pellet nochmals 2 x mit dem oben angegebenen Puffer gewaschen. Zu dem gewaschenen Pellet werden 0,6 ml einer 6 mM Lösung an Chlorphenolrot-β-galactosid gelöst in 0,1 M Natriumphosphatpuffer, pH 7,2, 0,9 % Nacl, 2 mM MgCl$_2$ und Lösung suspendiert und 5 min unter Schütteln inkubiert (37 ˚C). Danach wird wie oben abzentrifugiert. Vom Überstand werden 0,5 ml entnommen und davon die optische Dichte bei 578 nm bestimmt. Figur 2 der Zeichnung zeigt den Verlauf einer so hergestellten Eichkurve. Aufgetragen wurde die Digoxin-Konzentration des unverdünnten Serums (X-Achse) gegen die dazugehörige optische Dichte (Y-Achse).

## Beispiel 3

Die Bestimmung von Digoxin unter Verwendung eines Digoxin-Analogon als Festphase 1 :

### 1. Reagentien

Die Art und Herstellung von Reagentien und Festphase 2 entsprechen Beispiel 1 mit Ausnahme von Festphase 1, Kopplung von Digitoxin an Kaninchen-IgG.

Kaninchen-IgG wurde wie oben beschrieben gewonnen, mit Natrium-meta-perjodat oxidiert und an Kaninchen-IgG gekoppelt. Das Verfahren und die technischen Einzelheiten sind ausführlich von V. P. Butler jr. und J. P. Chen, Proc. Nat. Acad. Sci. US, 57, 7 (1967) sowie von T. W. Smith et al., Biochem. 9, 331-337 (1970) beschrieben. Abweichend von diesen beiden Vorschriften wurde als Trägerprotein nicht Serum-Albumin, sondern über DEAE-Cellulose aufgereinigtes Kaninchen-IgG verwendet.

5 mg Digitoxin derivatisiertes Kaninchen-IgG werden in 5 ml 0,1 M Natriumphosphatpuffer pH 7,2 plus 0,9 % Natriumchlorid gelöst. Dazu wird eine Lösung von Schaf-IgG (siehe Beispiel 1), gerichtet gegen Kaninchen-IgG (ca. 100 mg), aufgereinigt über Ammoniumsulfat-Fraktionierung und DEAE-Cellulose-Chromatographie, gelöst im gleichen Puffer, gegeben. Es bildet sich ein Niederschlag, der ca. 1 h bei Raumtemperatur stehengelassen wird. Danach wird der Niederschlag abzentrifugiert und anschließend in 10 ml des gleichen Puffers resuspendiert. Der Vorgang des Abzentrifugierens und Resuspendierens wird 4 x wiederholt. Abschließend wird der Niederschlag in 10 ml des obengenannten Puffers aufgenommen.

## 2. Bestimmung von Digoxin

Digoxin-Standards in Humanserum (entnommen aus dem Elisa-Kit von Boehringer Mannheim, Best. Nr. 199 656) wurden 1 : 7,5 mit 0,9 % NaCl-Lösung verdünnt. Zu 200 µl verdünntem Standard wurde 200 µl Binder (20 mU/ml), bestimmt mit ortho-nitrophenyl-β-D-galactosid), hergestellt wie unter 3.7 beschrieben, gegeben. Der Binder ist in folgendem Puffer gelöst :

0,1 M Natriumphosphat pH 7,2
0,2 % NaCl
2 mM MgCl$_2$
1 % Rinder-Serum-Albumin

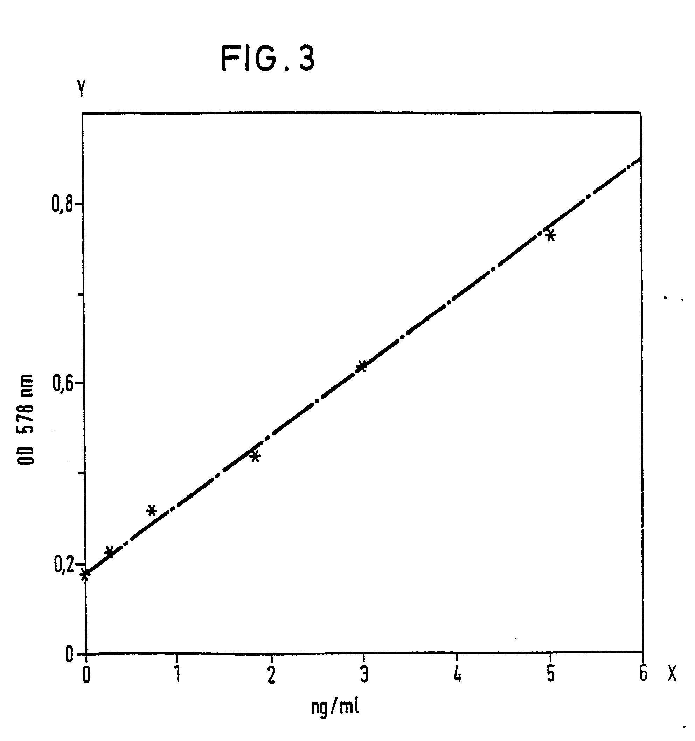

Standard und Binder werden genau 5 min bei 37 ˚C inkubiert. Danach erfolgt die Zugabe von 50 µl Festphase 1. Das Gemisch wird weitere 5 min unter Schütteln bei 37 ˚C inkubiert. Anschließend wird 1 min mit einer Eppendorf-Zentrifuge 54/4 zentrifugiert. Vom Überstand werden 300 µl entnommen, hinzugefügt wird 50 µl Festphase 2 und unter Schütteln bei 37 ˚C inkubiert. Der Ansatz wird nach Ablauf dieser 5 min zentrifugiert, der Überstand verworfen und das Pellet nochmals 2 x mit dem oben angegebenen Puffer gewaschen. Zu dem gewaschenen Pellet werden 0,6 ml einer 6 mM Lösung an Chlorphenolrot-β-galactosid gelöst in 0,1 M Natriumphosphatpuffer, pH 7,2, 0,9 % NaCl, 2 mM MgCl$_2$ und Lösung suspendiert und 5 min unter Schütteln (37 ˚C) inkubiert. Danach wird wie oben abzentrifugiert. Vom Überstand werden 0,5 ml entnommen und davon die optische Dichte bei 578 nm bestimmt. Figur 3 der Zeichnung zeigt den Verlauf einer so erhaltenen Eichkurve. Aufgetragen wurde die Digoxin-Konzentration des unverdünnten Serums (X-Achse) gegen die dazugehörige optische Dichte (Y-Achse).

## Patentansprüche

1. Verfahren zur immunchemischen quantitativen Bestimmung einer antigen wirksamen Substanz durch Inkubation der zu bestimmenden Substanz mit einem Binder, der aus einem markierten, gegen die zu bestimmende Substanz gerichteten ersten Antikörper oder einem Fab-oder Fab'-Fragment davon besteht, Inkubieren des Umsetzungsgemisches für eine bestimmte Zeitdauer mit an eine feste Phase gebunden vorliegender zu bestimmender Substanz oder einem Analogen davon, Abtrennung der flüssigen Phase, gegebenenfalls Waschen der festen Phase und Vereinigung der Waschflüssigkeit mit der flüssigen Phase, dadurch gekennzeichnet, daß man die flüssige Phase danach mit einem an eine feste Phase gebunden vorliegenden zweiten Antikörper, der gegen den Binder oder den Komplex aus Binder und zu bestimmender Substanz gerichtet ist und mit keiner der Komponenten des Komplexes kreuzreagiert, wenn er gegen den Komplex gerichtet ist, kontaktiert; die feste Phase abtrennt und gegebenenfalls wäscht und die daran gebundene Markierung mißt.

2. Verfahren nach Anspruch 1, dadurch gekennzeichnet, daß als antigen wirksame Substanz ein Hapten verwendet wird.

3. Verfahren nach Anspruch 1 oder 2, dadurch gekennzeichnet, daß als Marker ein Enzym verwendet wird.

4. Verfahren nach Anspruch 1, dadurch gekennzeichnet, daß die antigen wirksame Substanz kovalent, adsorptiv oder durch Präzipitation an den Träger gebunden ist.

5. Verfahren nach Anspruch 1, dadurch gekennzeichnet, daß der zweite Antikörper kovalent, adsorptiv oder durch Präzipitation an den Träger gebunden ist.

6. Verfahren nach einem der vorhergehenden Ansprüche, dadurch gekennzeichnet, daß der Binder im Überschuß, bezogen auf die in der Probe vorhandene zu bestimmende Substanz, eingesetzt wird.

7. Verfahren nach einem der vorhergehenden Ansprüche, dadurch gekennzeichnet, daß der Binder einen monoklonalen Antikörper oder ein Fab- bzw. Fab'-Fragment davon enthält.

8. Verfahren nach einem der vorhergehenden Ansprüche, dadurch gekennzeichnet, daß der zweite Antikörper monoklonal oder ein Fab/Fab'-Fragment davon ist.

9. Reagenz zur immunchemischen Bestimmung einer antigen wirksamen Substanz, gekennzeichnet durch einen Gehalt an

a) einem Binder, der aus einem markierten, gegen die zu bestimmende Substanz gerichteten ersten Antikörper oder einem Fab- bzw. Fab'-Fragment davon besteht,

b) an eine feste Phase gebundenem Antigen,

c) an eine feste Phase gebundenem zweiten Antikörper, der gegen den Binder oder den Komplex aus Binder und zu bestimmender Substanz gerichtet ist und

d) einem System zur Messung der Markierungssubstanz.

10. Reagenz nach Anspruch 9, dadurch gekennzeichnet, daß der Binder als Markierungssubstanz ein Enzym enthält.

**Claims**

1. Process for the immunochemical quantitative determination of an antigen-active substance by incubation of the substance to be determined with a binder, which consists of a labelled first antibody directed against the substance to be determined or a Fab or Fab' fragment thereof, incubation of the reaction mixture for a definite period of time with substance to be determined present bound to a solid phase or an analogue thereof, separation of the liquid phase, optional washing of the solid phase and combination of the wash liquid with the liquid phase, characterised in that one thereafter contacts the liquid phase with a second antibody, present bound to a solid phase, which is directed against the binder or the complex of binder and substance to be determined and does not cross-react with any of the components of the complex when it is directed against the complex, separates off the solid phase and possibly washes and measures the label bound thereon.

2. Process according to claim 1, characterised in that a hapten is used as antigen-active substance.

3. Process according to claim 1 to 2, characterised in that an enzyme is used as label.

4. Process according to claim 1, characterised in that the antigen-active substance is bound to the carrier covalently, adsorptively or by precipitation.

5. Process according to claim 1, characterised in that the second antibody is bound to the carrier covalently, adsorptively or by precipitation.

6. Process according to one of the preceding claims, characterised in that the binder is used in excess, referred to the substance to be determined present in the sample.

7. Process according to one of the preceding claims, characterised in that the binder contains a monoclonal antibody or a Fab or Fab' fragment thereof.

8. Process according to one of the preceding claims, characterised in that the second antibody is monoclonal or a Fab/Fab' fragment thereof.

9. Reagent for the immunochemical determination of an antigen-active substance, characterised by a content of

a) a binder which consists of a labelled first antibody directed against the substance to be determined or a Fab or Fab' fragment thereof,

b) antigen bound to a solid phase,

c) second antibody bound to a solid phase which is directed against the binder or the complex of binder and substance to be determined and

d) a system for the measurement of the labelling substance.

10. Reagent according to claim 9, characterised in that the binder contains an enzyme as labelling substance.

**Revendications**

1. Procédé pour la détermination immunochi- mique quantitative d'une substance à activité antigénique par incubation de la substance à déterminer avec un liant qui est constitué d'un premier anticorps dirigé contre la substance à déterminer, marqué, ou d'un fragment Fab ou Fab' de celui-ci, incubation du mélange de réaction pendant une durée déterminée avec la substance à déterminer présente liée à une phase solide ou avec un analogue de cette substance, séparation de la phase liquide, lavage éventuel de la phase solide et réunion du liquide de lavage avec la phase liquide, caractérisé en ce que l'on met ensuite en contact la phase liquide avec un deuxième anticorps présent lié à une phase solide, lequel deuxième anticorps est dirigé contre le liant ou contre le complexe formé du liant et de la substance à déterminer et ne réagit de façon croisée avec aucun des composants du complexe, lorsqu'il est dirigé contre le complexe, on sépare la phase solide et la lave éventuellement et on mesure le marquage qui y est lié.

2. Procédé selon la revendication 1, caractérisé en ce qu'on utilise, comme substance à activité antigénique, un haptène.

3. Procédé selon la revendication 1 ou 2, caractérisé en ce qu'on utilise comme marqueur une enzyme.

4. Procédé selon la revendication 1, caractérisé en ce que la substance à activité antigénique est liée au support de façon covalente, par adsorption ou par précipitation.

5. Procédé selon la revendication 1, caractérisé en ce que le deuxième anticorps est lié au support de façon covalente, par adsorption ou par précipitation.

6. Procédé selon l'une des revendications précédentes, caractérisé en ce que le liant est utilisé en excès par rapport à la substance à déterminer présente dans l'échantillon.

7. Procédé selon l'une des revendications précédentes, caractérisé en ce que le liant contient un anticorps monoclonal ou un fragment Fab ou respectivement Fab' de celui-ci.

8. Procédé selon l'une des revendications précédentes, caractérisé en ce que le deuxième anticorps est monoclonal ou est un fragment Fab/Fab' de celui-ci.

9. Réactif pour la détermination immunochimique d'une substance à activité antigénique caractérisé par une teneur en

a) un liant qui est constitué d'un premier anticorps dirigé contre la substance à déterminer, marqué ou d'un fragment Fab ou respectivement Fab' de celui-ci,

b) antigène lié à une phase solide,

c) un deuxième anticorps lié à une phase solide, lequel deuxième anticorps est dirigé contre le liant ou contre le complexe formé du liant et de la substance à déterminer et

d) un système pour la mesure de la substance de marquage.

10. Réactif selon la revendication 9, caractérisé en ce que le liant contient, en tant que substance de marquage, une enzyme.

# FIG.1

Regression
y = A * x + B
A =  .390
B =  .432
R =  .9990
N =  6

# FIG.2

# FIG.3